(19) Europäisches Patentamt
European Patent Office
Office européen des brevets

(11) **EP 4 566 568 A1**

(12) **EUROPÄISCHE PATENTANMELDUNG**

(43) Veröffentlichungstag:
**11.06.2025   Patentblatt 2025/24**

(21) Anmeldenummer: 24223902.8

(22) Anmeldetag: **29.11.2018**

(51) Internationale Patentklassifikation (IPC):
*A61C 13/00* (2006.01)     *A61C 13/083* (2006.01)
*A61C 5/77* (2017.01)

(52) Gemeinsame Patentklassifikation (CPC):
**A61C 5/77; A61C 13/0006; A61C 13/083;
C04B 35/486;** C04B 2235/3206; C04B 2235/3208;
C04B 2235/3225; C04B 2235/3229;
C04B 2235/6562; C04B 2235/6565;
C04B 2235/6567; C04B 2235/6581;
C04B 2235/6583; C04B 2235/6586;
C04B 2235/661;                          (Forts.)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(62) Dokumentnummer(n) der früheren Anmeldung(en)
nach Art. 76 EPÜ:
**18209308.8 / 3 659 548**

(71) Anmelder: **Ivoclar Vivadent AG**
**9494 Schaan (LI)**

(72) Erfinder:
• **Rothbrust, Frank**
**6822 Röns (AT)**
• **Jiang, Bo**
**9470 Werdenberg (CH)**

• **Jussel, Rudolf**
**6800 Feldkirch (AT)**
• **Ritzberger, Christian**
**9472 Grabs (CH)**

(74) Vertreter: **Uexküll & Stolberg
Partnerschaft von
Patent- und Rechtsanwälten mbB
Beselerstraße 4
22607 Hamburg (DE)**

Bemerkungen:
•Diese Anmeldung ist am 31-12-2024 als
Teilanmeldung zu der unter INID-Code 62 erwähnten
Anmeldung eingereicht worden.
•Die Patentansprüche wurden nach dem
Anmeldetag / dem Tag des Eingangs der
Teilanmeldung eingereicht (R. 68(4) EPÜ)

(54) **VERFAHREN ZUR HERSTELLUNG EINER DENTALEN RESTAURATION**

(57)     Die Erfindung betrifft ein Verfahren zur Herstellung einer dentalen Restauration, bei dem ein Oxidkeramikmaterial

(a) einer ersten Wärmebehandlung unterworfen wird,
(b) einer zweiten Wärmebehandlung unterworfen wird
und
(c) abgekühlt wird,
wobei die Wärmebehandlung in Schritt (a) bei niedrigerem Druck als die Wärmebehandlung in Schritt (b) erfolgt.

**EP 4 566 568 A1**

**(Forts. nächste Seite)**

(52) Gemeinsame Patentklassifikation (CPC): (Forts.)
C04B 2235/77; C04B 2235/9653;
C04B 2235/9661; C04B 2235/9669;
C04B 2237/348; C04B 2237/58

## Beschreibung

Die vorliegende Erfindung betrifft ein Verfahren, welches ausgehend von einem Oxidkeramikmaterial die Herstellung einer dentalen Restauration mit hervorragenden Eigenschaften in kurzer Zeit ermöglicht. Die Erfindung betrifft auch die Verwendung eines Oxidkeramikmaterials zur Herstellung einer dentalen Restauration mittels des erfindungsgemäßen Verfahrens.

Zur Herstellung vollanatomischer Dentalrestaurationen werden häufig keramische Materialien wie Oxidkeramiken eingesetzt. Diese bieten eine hohe klinische Sicherheit, sind üblicherweise metallfrei, können auch bei minimalinvasiven Präparationen eingesetzt werden und sind preislich im Vergleich zu anderen metallfreien Restaurationen sehr attraktiv. Nachteilig sind jedoch die zahlreichen Arbeitsschritte, die meist zur Herstellung solcher Restaurationen erforderlich sind.

Üblicherweise werden die Restaurationen aus vorgesinterten Rohlingen herausgefräst oder geschliffen, gegebenenfalls eingefärbt, durch thermische Behandlung dichtgesintert und schließlich gegebenenfalls weiter eingefärbt, glasiert und/oder poliert.

Konventionelle Sinterverfahren für Dentalkeramiken beinhalten ein langsames Aufheizen auf eine Maximaltemperatur, bei der das eingesetzte Oxidkeramikmaterial dichtgesintert wird. Bedingt durch die geringe Aufheizgeschwindigkeit dauert ein solcher Sinterprozess typischerweise deutlich mehr als 4 Stunden und trägt dadurch erheblich zu einer vor allem bei Chairside-Behandlungen unbefriedigend großen Dauer des Produktionszyklus von Dentalkeramiken bei.

Ansätze zur Beschleunigung des Sinterprozesses durch Erhöhung der Aufheizgeschwindigkeit sind grundsätzlich bekannt.

So beschreibt EP 2 098 188 A1 einen Dentalofen und ein Verfahren zum Sintern von Dentalmaterialien, bei dem der Ofen in einer ersten Aufheizperiode mit einer schnellen Aufheizrate von mehr als 50 K/min auf eine Vorsintertemperatur von mindestens 1000°C aufgeheizt wird.

EP 2 101 133 A1 beschreibt einen Sinterofen und ein Verfahren zum Sintern von Dentalpräparaten, bei dem die Dentalpräparate entlang einer Sinterstrecke bewegt und dabei verschiedenen Temperaturen ausgesetzt werden. Dabei können in einem ersten Abschnitt hohe Aufheizraten von 300 K/min oder mehr eingesetzt werden.

WO 2012/057829 A2 beschreibt ein Verfahren zum schnellen Sintern von Keramik unter Verwendung von elektromagnetischer Induktion oder eines Plasmas.

WO 2015/091744 A1 beschreibt ein Verfahren zur Planung einer Sinterung eines Zahnersatzteils, bei dem in Abhängigkeit von bestimmten Geometrie- und Materialparametern des herzustellenden Zahnersatzteils ein Temperaturprofil für die Wärmebehandlung des Zahnersatzteils automatisch mittels eines Computers bestimmt wird. Dabei wird für die Sinterung bestimmter Zahnersatzteile eine Heizrate zwischen 100 K/min und 400 K/min verwendet.

WO 2015/121364 A1 beschreibt einen Sinterofen für Dentalbauteile mit einer Aufheizvorrichtung, die im Nutzbereich eine Aufheizrate von mindestens 200 K/min ermöglicht.

Auch die Sinterung von Dentalmaterialien unter Schutzgas oder im Vakuum ist bekannt.

WO 2011/020688 A1 beschreibt eine Vorrichtung zum sauerstofffreien Sintern von Metall oder Keramik in der Dentaltechnik unter Schutzgas.

EP 2 703 760 A1 beschreibt einen Dentalofen zum Sintern eines Zahnersatzes, dessen Heizraum entweder mittels einer konventionellen Verschlussvorrichtung wie etwa einer Tür oder mittels eines Aufsatzes mit einem Vakuumbehälter verschließbar ist und dadurch wahlweise zum normalen Sintern oder zum Vakuumsintern eingesetzt werden kann.

Es hat sich jedoch gezeigt, dass die bekannten Ansätze zur Beschleunigung des Sinterprozesses zu Keramikmaterialien führen, deren Eigenschaften insbesondere in optischer Hinsicht den hohen Anforderungen im Dentalbereich nicht genügen.

Der Erfindung liegt daher die Aufgabe zugrunde, ein Verfahren zur Herstellung einer dentalen Restauration bereitzustellen, mit dem in kurzer Zeit durch Sintern von Oxidkeramikmaterial dentale Restauration mit hervorragenden mechanischen und insbesondere optischen Eigenschaften hergestellt werden können.

Diese Aufgabe wird erfindungsgemäß gelöst durch das Verfahren zur Herstellung einer dentalen Restauration nach den Absätzen 1 bis 15. Gegenstand der Erfindung ist auch die Verwendung eines Oxidkeramikmaterials zur Herstellung einer dentalen Restauration nach Absatz 16.

Das erfindungsgemäße Verfahren zur Herstellung einer dentalen Restauration zeichnet sich dadurch aus, dass ein Oxidkeramikmaterial

(a) einer ersten Wärmebehandlung unterworfen wird,
(b) einer zweiten Wärmebehandlung unterworfen wird und
(c) abgekühlt wird,

wobei die Wärmebehandlung in Schritt (a) bei niedrigerem Druck als Schritt (b) erfolgt.

Es wurde überraschend gefunden, dass das erfindungsgemäße Verfahren eine sehr schnelle Sinterung von Oxidkeramiken zu dentalen Restaurationen ermöglicht, die gute mechanische Eigenschaften und insbesondere eine hohe Dichte aufweisen und zugleich auch in ästhetischer Hinsicht die hohen Anforderungen an dentale Restaurationen erfüllen und die optischen Eigenschaften von natürlichem Zahnmaterial ausgezeichnet imitieren können.

Bei dem in Schritt (a) eingesetzten Oxidkeramikmaterial handelt es sich üblicherweise um ein nicht dichtgesintertes und insbesondere um ein vorgesintertes Oxidkeramikmaterial. Üblicherweise weist das eingesetzte Oxidkeramikmaterial eine relative Dichte im Bereich von 30 bis 90%, insbesondere im Bereich von 40 bis 80% und bevorzugt im Bereich von 50 bis 70%, jeweils bezogen auf die Reindichte des Oxidkeramikmaterials, auf.

Die relative Dichte ist dabei das Verhältnis der scheinbaren Dichte des Oxidkeramikmaterials zur Reindichte des Oxidkeramikmaterials.

Die scheinbare Dichte des Oxidkeramikmaterials kann mittels der Immersionsmethode gemäß ISO 18754 aus der Masse des trockenen Probe ($m_t$), der scheinbaren Masse der in einer Immersionsflüssigkeit untergetauchten Probe ($m_i$) und der Dichte der Immersionsflüssigkeit ($\rho_i$) nach der Formel

$$\rho = \frac{m_t}{m_t - m_i} \times \rho_i$$

berechnet werden. Als Immersionsflüssigkeit wird vorzugsweise Tetrachlorkohlenstoff ($CCl_4$) eingesetzt.

Die Bestimmung der Reindichte des Oxidkeramikmaterials erfolgt, indem das Oxidkeramikmaterial auf ein Pulver mit einer mittleren Teilchengröße von 10 bis 30 $\mu$m, insbesondere von 20 $\mu$m, bezogen auf die Anzahl der Teilchen, gemahlen und die Dichte des Pulvers mittels Pyknometer ermittelt wird. Die Bestimmung der Teilchengröße kann beispielsweise mit dem CILAS® Particle Size Analyzer 1064 der Firma Quantachrome GmbH & Co. KG mittels Laserbeugung nach ISO 13320 (2009) durchgeführt werden.

In Schritt (a) wird das Oxidkeramikmaterial vorzugsweise auf eine Temperatur aufgeheizt, die im Bereich von 1100 bis 1600°C, insbesondere im Bereich von 1200 bis 1500°C, bevorzugt im Bereich von 1250 bis 1450°C und weiter bevorzugt im Bereich von 1300 bis 1400°C liegt und am meisten bevorzugt etwa 1350°C beträgt. Es ist weiter bevorzugt, dass das Oxidkeramikmaterial am Ende von Schritt (a) eine relative Dichte im Bereich von 90 bis 97%, insbesondere im Bereich von 93 bis 96% und bevorzugt eine relative Dichte von etwa 95%, jeweils bezogen auf die Reindichte des Oxidkeramikmaterials, aufweist.

Vorzugsweise wird das Oxidkeramikmaterial in Schritt (a) mit einer Heizrate im Bereich von 10 bis 500 K/min, insbesondere 50 bis 250 K/min und bevorzugt 100 bis 200 K/min aufgeheizt. In einer bevorzugten Ausführungsform wird das Oxidkeramikmaterial zunächst mit einer Heizrate von 50 bis 500 K/min, insbesondere 100 bis 250 K/min und bevorzugt 150 bis 200 K/min auf eine Temperatur aufgeheizt, die 100 bis 700 K, insbesondere 200 bis 450 K und vorzugsweise etwa 350 K unterhalb der maximalen in Schritt (a) erreichten Temperatur liegt, und anschließend mit einer Heizrate von 10 bis 200 K/min, insbesondere 25 bis 150 K/min und bevorzugt 50 bis 100 K/min weiter aufgeheizt.

Die Wärmebehandlung in Schritt (a) erfolgt erfindungsgemäß bei niedrigerem Druck als die Wärmebehandlung in Schritt (b). Bevorzugt erfolgt die Wärmebehandlung in Schritt (a) bei einem Druck von weniger als 200 mbar, vorzugsweise weniger als 100 mbar und besonders bevorzugt weniger als 50 mbar, und insbesondere bei einem Druck im Bereich von 0,1 bis 200 mbar, vorzugsweise im Bereich von 1 bis 150 mbar und am besonders bevorzugt im Bereich von 50 bis 100 mbar.

Die Einstellung dieses Drucks kann bei Umgebungstemperatur erfolgen, bevor mit dem Aufheizen des Oxidkeramikmaterials begonnen wird. Alternativ kann das Oxidkeramikmaterial zunächst auf eine oberhalb der Umgebungstemperatur liegende Temperatur aufgeheizt werden, bevor der für Schritt (a) definierte Druck eingestellt wird. Diese Temperatur liegt vorzugsweise im Bereich von 20 bis 500°C und insbesondere im Bereich von 25 bis 100°C.

In Schritt (b) wird das Oxidkeramikmaterial vorzugsweise (b1) gegebenenfalls weiter aufgeheizt und (b2) bei einer, vorzugsweise konstanten, Temperatur im Bereich von 1100 bis 1700°C, insbesondere im Bereich von 1300 bis 1600°C und bevorzugt im Bereich von 1350 bis 1550°C und besonders bevorzugt bei einer Temperatur etwa 1500°C gehalten und gesintert. Dabei erfolgt das weitere Aufheizen in Schritt (b1) vorzugsweise mit einer Heizrate von 10 bis 200 K/min, insbesondere 25 bis 150 K/min und bevorzugt 50 bis 100 K/min. Das Halten in Schritt (b2) erfolgt vorzugsweise für 1 bis 60 Minuten, insbesondere 2 bis 30 Minuten, bevorzugt 3 bis 15 Minuten und besonders bevorzugt etwa 5 Minuten. Durch das Halten bei der entsprechenden Temperatur wird das Oxidkeramikmaterial typischerweise dichtgesintert. Danach weist das Oxidkeramikmaterial vorzugsweise eine relative Dichte von mindestens 97%, insbesondere mindestens 98%, bevorzugt mindestens 99% und am meisten bevorzugt mindestens 99,5%, jeweils bezogen auf die Reindichte des Oxidkeramikmaterials, auf.

Die Wärmebehandlung in Schritt (b) erfolgt erfindungsgemäß bei höherem Druck als die Wärmebehandlung in Schritt (a). Vorzugsweise wird die Wärmebehandlung in Schritt (b) bei einem Druck von mehr als 500 mbar und insbesondere bei Umgebungsdruck ausgeführt.

Bevorzugt erfolgt die Wärmebehandlung in Schritt (b) in einer sauerstoffhaltigen Atmosphäre. Als sauerstoffhaltige Atmosphäre kommen insbesondere Luft, mit Sauerstoff angereicherte Luft sowie Sauerstoff in Frage. Zur Einstellung einer solchen Atmosphäre kann die für die Wärmebehandlung verwendete Heizkammer mit Luft und/oder Sauerstoff gefüllt werden. In einer bevorzugten Ausführungsform wird die für die Wärmebehandlung verwendete Heizkammer während Schritt (b) diskontinuierlich oder vorzugsweise kontinuierlich mit einer sauerstoffhaltigen Atmosphäre, vorzugsweise Luft, mit Sauerstoff angereicherter Luft oder Sauerstoff, durchströmt, insbesondere mit einer Strömungsgeschwindigkeit von 0,1 bis 50 l/min, bevorzugt 1 bis 20 l/min und besonders bevorzugt 4 bis 8 l/min.

Außerdem ist es bevorzugt, dass das Oxidkeramikmaterial in Schritt (a) auf eine Temperatur aufgeheizt wird, die 0 bis 500 K, insbesondere 10 bis 250 K, bevorzugt 50 bis 200 K und besonders bevorzugt 100 bis 150 K unterhalb der Temperatur oder des Temperaturbereichs liegt, bei der/dem das Oxidkeramikmaterial in Schritt (b) gehalten wird.

Anschließend wird in Schritt (c) das Oxidkeramikmaterial abgekühlt. Vorzugsweise wird das Oxidkeramikmaterial auf eine Temperatur abgekühlt, die im Bereich von 20 bis 1300°C, insbesondere im Bereich von 100 bis 1250°C und bevorzugt im Bereich von 1000 bis 1200°C liegt. Nach Erreichen einer solchen Temperatur kann das Oxidkeramikmaterial aus der Heizkammer entfernt werden. Vorzugsweise erfolgt das Abkühlen mit einer Abkühlrate von 50 bis 200 K/min, insbesondere 75 bis 175 K/min und bevorzugt 100 bis 150 K/min.

Das nach dem erfindungsgemäßen Verfahren erhaltene Oxidkeramikmaterial weist vorzugsweise eine zahlenmittlere Korngröße im Bereich von 1 nm bis 1000 nm, insbesondere von 10 nm bis 800 nm und bevorzugt von 100 nm bis 600 nm auf. Die zahlenmittlere Korngröße kann insbesondere nach dem Linienschnittverfahren gemäß DIN EN 623-3 oder ASTM E 112 bestimmt werden, wobei der ermittelte Wert zur Umrechnung auf die reale zahlenmittlere Korngröße im dreidimensionalen Mikrogefüge nach M. I. Mendelson, J. Am. Ceram. Soc. 1969, 52(8), 443-446 mit einer Proportionalitätskonstante von 1,56 multipliziert wird.

Das erfindungsgemäße Verfahren eignet sich für verschiedene Arten von Oxidkeramikmaterialien. Oxidkeramikmaterialien sind allgemein hochkristalline Keramikmaterialien, die auf Oxidverbindungen basieren und allenfalls einen sehr geringen Anteil an Glasphase aufweisen. Typische Oxidkeramikmaterialien basieren auf $ZrO_2$, $Al_2O_3$, $TiO_2$, MgO, Kombinationen, Mischkristallen oder Kompositen davon, insbesondere $ZrO_2/Al_2O_3$ (ZTA), $Al_2O_3/ZrO_2$ (ATZ) oder $ZrO_2$/Spinell, wobei Spinell vorzugsweise Sr-Spinell, Mg-Spinell, La-Spinell und/oder Ce-Spinell ist. Oxidkeramikmaterialien auf Basis von $ZrO_2$ und/oder $Al_2O_3$ sind erfindungsgemäß bevorzugt.

Besonders bevorzugt sind Oxidkeramikmaterialien auf Basis von Zirkonoxid und insbesondere auf Basis von polykristallinem tetragonalem Zirkonoxid (TZP). Ganz besonders bevorzugt sind Oxidkeramikmaterialien auf Basis von Zirkonoxid, bei denen das Zirkonoxid mit $Y_2O_3$, $La_2O_3$, $CeO_2$, MgO und/oder CaO stabilisiert ist und vorzugsweise mit 2 bis 12 Mol-%, insbesondere 3 bis 5 Mol-%, dieser Oxide, bezogen auf den Gehalt an Zirkonoxid, stabilisiert ist.

Es ist weiter bevorzugt, dass das Oxidkeramikmaterial eingefärbt ist. Hierunter wird erfindungsgemäß ein Oxidkeramikmaterial verstanden, das mit einem oder mehreren färbenden Elementen versetzt ist. Beispiele für geeignete färbende Elemente sind Fe, Mn, Cr, Pr, Tb, Er, Yb, Ce, Co, Ni, Nd, Cu und Bi. Besonders bevorzugt umfasst das Oxidkeramikmaterial mindestens zwei Schichten, die sich in ihrer Farbe unterscheiden.

Im Sinne der vorliegenden Anmeldung beziehen sich die Begriffe "Farbe" und "eingefärbt" auf die Farbe, Helligkeit und/oder Transluzenz eines Materials.

"Transluzenz" ist die Lichtdurchlässigkeit eines Materials, Körpers oder einer Schicht, d.h. das Verhältnis von durchgelassener zu eingestrahlter Lichtintensität.

Farben können auch durch die Farbkoordinaten L*, a* und b* im L*a*b*-Farbraum oder durch einen in der Dentalindustrie üblicherweise verwendeten Farbcode charakterisiert werden. Im L*a*b*-Farbraum beschreibt der Wert L* die Helligkeit einer Farbe mit Werten von 0 (schwarz) bis 100 (weiß), der Wert a* den Grün- oder Rotanteil einer Farbe, wobei negative Werte für Grün und positive Werte für Rot stehen, und der Wert b* den Blau- oder Gelbanteil einer Farbe, wobei negative Werte für Blau und positive Werte für Gelb stehen. Beispiele für in der Dentalindustrie übliche Farbcodes sind Vitapan classical® und Vita 3D Master®, beide von der VITA Zahnfabrik H. Rauter GmbH & Co. KG, und Chromascop® von der Ivoclar Vivadent AG. Die Transluzenz kann durch den Kontrastwert CR charakterisiert werden, wobei 0% vollständig transparent und 100% vollständig opak bedeutet.

Üblicherweise erfolgen die Bestimmung der Farbkoordinaten L*, a* und b* nach DIN 5033 und DIN 6174 und die Bestimmung der Transluzenz nach BS 5612. Die entsprechenden Messungen können insbesondere mittels eines Spektrophotometers vom Typ CM-3700d (Konica-Minolta) durchgeführt werden. Hierzu werden für die Messungen Probenkörper eingesetzt, die mit Diamantpartikeln (Partikelgröße 15-20 $\mu$m) beidseitig nass beschliffen wurden, um eine finale Probendicke von 2,00 $\pm$ 0,025 mm zu erhalten.

Vorzugsweise liegen die Farbe oder die Farben der erfindungsgemäß erhaltenen dentalen Restauration im Bereich der Farben natürlicher Zähne. Besonders bevorzugt weisen die erfindungsgemäß erhaltenen dentalen Restauration einen L*-Wert im Bereich von 50 bis 100, insbesondere im Bereich von 80 bis 97, einen a*-Wert im Bereich von -10 bis 10, insbesondere im Bereich von -1 bis 5, einen b*-Wert im Bereich von 0 bis 50, insbesondere im Bereich von 1 bis 20, und/oder einen CR-Wert im Bereich von 50 bis 100%, insbesondere im Bereich von 75 bis 99%, auf.

Das erfindungsgemäße Verfahren eignet sich in besonderer Weise für die Herstellung von dentalen Restaurationen. Besonders bevorzugte dentale Restaurationen sind Brücken, Inlays, Onlays, Kronen, Veneers, Schalen und Abutments. Ganz besonders eignet sich das erfindungsgemäße Verfahren zur Herstellung von dentalen Restaurationen, insbesondere Brücken, die zwei oder mehr Glieder umfassen.

Die Erfindung betrifft auch die Verwendung eines Oxidkeramikmaterials zur Herstellung einer dentalen Restauration, bei der das Oxidkeramikmaterial

(a) einer ersten Wärmebehandlung unterworfen wird,
(b) einer zweiten Wärmebehandlung unterworfen wird und
(c) abgekühlt wird,

wobei die Wärmebehandlung in Schritt (a) bei niedrigerem Druck als die Wärmebehandlung in Schritt (b) erfolgt.

Bevorzugte Ausführungsformen der Verwendung sind wie oben für das erfindungsgemäße Verfahren beschrieben.

Im Folgenden werden bevorzugte Ausführungsformen der Erfindung in Form nummerierter Absätze beschrieben:

1. Verfahren zur Herstellung einer dentalen Restauration, bei dem ein Oxidkeramikmaterial

(a) einer ersten Wärmebehandlung unterworfen wird,
(b) einer zweiten Wärmebehandlung unterworfen wird und
(c) abgekühlt wird,

wobei die Wärmebehandlung in Schritt (a) bei niedrigerem Druck als die Wärmebehandlung in Schritt (b) erfolgt.

2. Verfahren nach Absatz 1, bei dem in Schritt (a) das Oxidkeramikmaterial auf eine Temperatur aufgeheizt wird, die im Bereich von 1100 bis 1600°C, insbesondere im Bereich von 1200 bis 1500°C, bevorzugt im Bereich von 1250 bis 1450°C und weiter bevorzugt im Bereich von 1300 bis 1400°C liegt und am meisten bevorzugt etwa 1350°C beträgt.

3. Verfahren nach Absatz 1 oder 2, bei dem das Oxidkeramikmaterial in Schritt (a) mit einer Heizrate im Bereich von 10 bis 500 K/min, insbesondere 50 bis 250 K/min und bevorzugt 100 bis 200 K/min aufgeheizt wird.

4. Verfahren nach einem der Absätze 1 bis 3, bei dem die Wärmebehandlung in Schritt (a) bei einem Druck von weniger als 200 mbar, vorzugsweise weniger als 100 mbar und besonders bevorzugt weniger als 50 mbar und insbesondere bei einem Druck im Bereich von 0,1 bis 200 mbar, vorzugsweise im Bereich von 1 bis 150 mbar und besonders bevorzugt im Bereich von 50 bis 100 mbar erfolgt.

5. Verfahren nach einem der Absätze 1 bis 4, bei dem in Schritt (b) das Oxidkeramikmaterial (b1) gegebenenfalls weiter aufgeheizt wird und (b2) bei einer, vorzugsweise konstanten, Temperatur im Bereich von 1100 bis 1700°C, insbesondere im Bereich von 1300 bis 1600°C und bevorzugt im Bereich von 1350 bis 1550°C und besonders bevorzugt bei einer Temperatur von etwa 1500°C gehalten und gesintert wird.

6. Verfahren nach Absatz 5, bei dem das Halten in Schritt (b2) für 1 bis 60 Minuten, insbesondere 2 bis 30 Minuten, bevorzugt 3 bis 15 Minuten und besonders bevorzugt etwa 5 Minuten erfolgt.

7. Verfahren nach einem der Absätze 1 bis 6, bei dem die Wärmebehandlung in Schritt (b) bei einem Druck von mehr als 500 mbar und insbesondere bei Umgebungsdruck erfolgt.

8. Verfahren nach einem der Absätze 1 bis 7, bei dem die Wärmebehandlung in Schritt (b) in einer sauerstoffhaltigen Atmosphäre und insbesondere in Luft, mit Sauerstoff angereicherter Luft oder Sauerstoff erfolgt.

9. Verfahren nach Absatz 8, bei dem während Schritt (b) die Heizkammer diskontinuierlich oder vorzugsweise kontinuierlich mit einer sauerstoffhaltigen Atmosphäre, vorzugsweise Luft, mit Sauerstoff angereicherter Luft oder Sauerstoff, durchströmt wird, insbesondere mit einer Strömungsgeschwindigkeit von 0,1 bis 50 l/min, bevorzugt 1 bis 20 l/min und besonders bevorzugt 4 bis 8 l/min.

10. Verfahren nach einem der Absätze 5 bis 9, bei dem das Oxidkeramikmaterial in Schritt (a) auf eine Temperatur aufgeheizt wird, die 0 bis 500 K, insbesondere 10 bis 250 K, bevorzugt 50 bis 200 K und besonders bevorzugt 100 bis 150 K unterhalb der Temperatur oder des Temperaturbereichs liegt, bei der/dem das Oxidkeramikmaterial in Schritt (b) gehalten wird.

11. Verfahren nach einem der Absätze 1 bis 10, bei dem das Oxidkeramikmaterial in Schritt (c) auf eine Temperatur abgekühlt wird, die im Bereich von 20 bis 1300°C, insbesondere im Bereich von 100 bis 1250°C und bevorzugt im Bereich von 1000 bis 1200°C liegt.

12. Verfahren nach einem der Absätze 1 bis 11, bei dem das Oxidkeramikmaterial auf Zirkonoxid und insbesondere auf polykristallinem tetragonalen Zirkonoxid (TZP) basiert.

13. Verfahren nach Absatz 12, bei dem das Zirkonoxid mit $Y_2O_3$, $CeO_2$, MgO und/oder CaO stabilisiert ist und vorzugsweise mit 2 bis 12 Mol-%, insbesondere 3 bis 5 Mol-%, dieser Oxide, bezogen auf den Gehalt an Zirkonoxid, stabilisiert ist.

14. Verfahren nach einem der Absätze 1 bis 13, bei dem das Oxidkeramikmaterial eingefärbt ist und vorzugsweise mindestens zwei Schichten umfasst, die sich insbesondere in ihrer Farbe unterscheiden.

15. Verfahren nach einem der Absätze 1 bis 14, bei dem die dentale Restauration eine Brücke, ein Inlay, ein Onlay, eine Krone, ein Veneer, eine Schale oder ein Abutment ist und vorzugsweise zwei oder mehr Glieder umfasst.

16. Verwendung eines Oxidkeramikmaterials zur Herstellung einer dentalen Restauration, bei der das Oxidkeramikmaterial

    (a) einer ersten Wärmebehandlung unterworfen wird,
    (b) einer zweiten Wärmebehandlung unterworfen wird und
    (c) abgekühlt wird,

wobei die Wärmebehandlung in Schritt (a) bei niedrigerem Druck als die Wärmebehandlung in Schritt (b) erfolgt.

Die Erfindung wird im Folgenden anhand von Beispielen näher erläutert.

**Beispiele**

**Allgemeine Verfahrensweise: Herstellung von Prüfkörpern**

Aus kommerziell erhältlichen Blöcken oder Scheiben aus Oxidkeramik wurden durch Trockensägen mit einer Diamantsäge Prüfkörper mit einer Höhe von 17 mm, einer Breite von 15,5 mm und einer Dicke von 2,5 bis 3,5 mm hergestellt. Nach dem Sägen wurden die Prüfkörper bei 80°C für 2 h in einem Trockenschrank getrocknet.

**Beispiel 1A**

Ein gemäß der allgemeinen Verfahrensweise aus kommerziell erhältlichen Oxidkeramik-Blöcken auf Basis von Zirkonoxid mit 3 Mol-% $Y_2O_3$ (IPS e.max ZirCAD LT BL, Ivoclar Vivadent) erhaltener Prüfkörper wurde in einem Sinterofen mit SiC-Heizelement gesintert. Dazu wurde der Prüfkörper bei Raumtemperatur in die Heizkammer des Sinterofens eingebracht, die Heizkammer verschlossen und in der Heizkammer ein partielles Vakuum mit einem finalen Druck von etwa 50 bis 100 mbar erzeugt. Der Prüfkörper wurde mit einer Heizrate von etwa 180 K/min auf eine Temperatur von etwa 1000°C und weiter mit einer Heizrate von etwa 80 K/min auf eine Temperatur von etwa 1350°C aufgeheizt. Bei Erreichen dieser Temperatur wurde die Heizkammer mit Frischluft geflutet und anschließend kontinuierlich mit einer Strömungsgeschwindigkeit von etwa 5 l/min mit Frischluft durchströmt, während der Prüfkörper mit einer Heizrate von etwa 80 K/min weiter auf eine Temperatur von 1500°C aufgeheizt, bei dieser Temperatur etwa 5 Minuten lang gehalten und dann mit einer Abkühlrate von etwa 140 K/min auf eine Temperatur von etwa 1100°C abgekühlt wurde. Anschließend wurde die Heizkammer geöffnet. Die Gesamtdauer des Sinterprozesses betrug etwa 19,5 min.

**Beispiel 1B (Vergleich)**

Beispiel 1A wurde wiederholt, wobei jedoch kein Vakuum erzeugt wurde, sondern die Heizkammer über die gesamte Dauer des Sinterprozesses kontinuierlich mit einer Strömungsgeschwindigkeit von etwa 5 l/min mit Frischluft durchströmt wurde.

**Beispiel 1C (Vergleich)**

Ein gemäß der allgemeinen Verfahrensweise aus kommerziell erhältlichen Oxidkeramik-Blöcken auf Basis von Zirkonoxid mit 3 Mol-% $Y_2O_3$ IPS e.max ZirCAD LT BL, Ivoclar Vivadent) erhaltener Prüfkörper wurde in einem Sinterofen vom Typ Programat CS4 (Ivoclar Vivadent) mit dem Programm P2 ohne automatische Vortrocknung gesintert. Dabei wurde der Prüfkörper mit einer Heizrate von etwa 130 K/min von Raumtemperatur auf etwa 900°C, weiter mit einer Heizrate von etwa 50 K/min auf etwa 1035°C und schließlich mit einer Heizrate von etwa 15 K/min auf etwa 1460°C aufgeheizt. Bei dieser Temperatur wurde der Prüfkörper etwa 6 min lang gehalten und dann mit einer Abkühlrate von etwa 70 K/min auf eine Temperatur von etwa 1200°C abgekühlt. Anschließend wurde die Heizkammer geöffnet. Die Gesamtdauer des Sinterprozesses betrug etwa 47 min.

Die Dichte, Transluzenz und Farbkoordinaten der in den Beispielen 1A-C erhaltenen Oxidkeramikmaterialien sind in Tabelle 1 wiedergegeben.

Tabelle 1

| Beispiel | Sinterdauer [min] | Dichte [g/cm$^3$] | CR [%] | L* | a* | b* |
|---|---|---|---|---|---|---|
| 1A | 19,5 | 6, 065 | 88, 99 | 95,87 | -0,37 | 1, 75 |
| 1B (Vergleich) | 19,5 | 6,066 | 92,54 | 96,14 | -0,31 | 1, 81 |
| 1C (Vergleich) | 47 | 6, 068 | 88,53 | 95,79 | -0,27 | 1,96 |

Danach ermöglicht es das erfindungsgemäße Verfahren gemäß Beispiel 1A, bei sehr kurzer Sinterdauer von unter 20 min eine mit den Beispielen 1B und 1C vergleichbaren Dichte zu gelangen und dabei zugleich eine Transluzenz zu erhalten, die mit der bei langsamer Sinterung gemäß Beispiel 1C erhaltenen Transluzenz vergleichbar und deutlich höher als die bei schneller Sinterung gemäß Beispiel 1B erhaltene Transluzenz ist.

**Beispiel 2**

Gemäß der allgemeinen Verfahrensweise aus kommerziell erhältlichen Oxidkeramik-Blöcken auf Basis von Zirkonoxid mit 3 Mol-% $Y_2O_3$ (IPS e.max ZirCAD LT BL B45 oder IPS e.max ZirCAD LT A3 B45, Ivoclar Vivadent) bzw. aus kommerziell erhältlichen Oxidkeramik-Scheiben auf Basis von Zirkonoxid (Zenostar MT 3, Wieland Dental + Technik) erhaltene Prüfkörper wurden jeweils in einem Sinterofen mit $MoSi_2$-Heizelement gesintert. Dazu wurden die Prüfkörper bei Raumtemperatur in die Heizkammer des Sinterofens eingebracht, die Heizkammer verschlossen und in der Heizkammer ein partielles Vakuum mit einem finalen Druck von <50 mbar erzeugt. Die Prüfkörper wurden mit einer Heizrate von etwa 180 K/min auf eine Temperatur von etwa 1050°C und weiter mit einer Heizrate von etwa 80 K/min auf eine Temperatur von etwa 1500°C aufgeheizt. Gemäß der Tabelle 2 wurde die Heizkammer bei Erreichen einer Temperatur von 1350, 1400, 1450 bzw. 1500°C mit Frischluft geflutet und anschließend während des restlichen Sinterprozesses kontinuierlich mit einer Strömungsgeschwindigkeit von etwa 5 l/min mit Frischluft durchströmt. Bei einer Temperatur von etwa 1500°C wurden die Prüfkörper etwa 5 Minuten lang gehalten und dann mit einer Abkühlrate von etwa 140 K/min auf eine Temperatur von etwa 1100°C abgekühlt. Anschließend wurde die Heizkammer geöffnet.

Zum Vergleich wurde die obige Verfahrensweise wiederholt, wobei jedoch kein Vakuum erzeugt wurde, sondern die Heizkammer über die gesamte Dauer des Sinterprozesses kontinuierlich mit einer Strömungsgeschwindigkeit von etwa 5 l/min mit Frischluft durchströmt wurde.

Die Dichte, Transluzenz und Farbkoordinaten der jeweils erhaltenen Oxidkeramikmaterialien sind in Tabelle 2 wiedergegeben.

Tabelle 2

| Material | Vakuum bis [°C] | Dichte [g/cm$^3$] | CR [%] | L* | a* | b* |
|---|---|---|---|---|---|---|
| IPS e.max ZirCAD LT BL B45 | 1350 | 6, 072 | 91,06 | 95,48 | -0,39 | 2,70 |
| | 1400 | 6, 075 | 89, 98 | 95,44 | -0,41 | 2,95 |
| | 1450 | 6, 077 | 89, 04 | 95,38 | -0, 48 | 3, 01 |
| | 1500 | 6,075 | 88,05 | 94,95 | -0,47 | 3,25 |
| | kein Vakuum* | 6, 070 | 92,35 | 96, 06 | -0,25 | 1,85 |

(fortgesetzt)

| Material | Vakuum bis [°C] | Dichte [g/cm³] | CR [%] | L* | a* | b* |
|---|---|---|---|---|---|---|
| IPS e.max ZirCAD LT A3 B45 | 1350 | 6, 079 | 96, 66 | 82,78 | 4,76 | 17,89 |
| | 1400 | 6, 080 | 96, 96 | 83, 12 | 4,55 | 17,45 |
| | 1450 | 6, 081 | 94,14 | 81,31 | 5,41 | 18,34 |
| | 1500 | 6, 078 | 97, 62 | 83,59 | 3, 80 | 16,37 |
| | kein Vakuum* | 6, 070 | 97,29 | 84,82 | 3,77 | 16,34 |
| Zenostar MT 3 | 1350 | 6, 041 | 98, 83 | 86,13 | 1,53 | 14,73 |
| | 1400 | 6,041 | 98,34 | 87,21 | 1,14 | 14,11 |
| | 1450 | 6, 048 | 96, 93 | 83,93 | 2,23 | 15,91 |
| | 1500 | 6, 044 | 98,71 | 86, 16 | 1, 48 | 15, 65 |
| | kein Vakuum* | 6, 033 | 99, 50 | 86,96 | 1,27 | 14,45 |
| * (Vergleich) | | | | | | |

**Beispiel 3**

Gemäß der allgemeinen Verfahrensweise aus kommerziell erhältlichen Oxidkeramik-Blöcken auf Basis von Zirkonoxid mit 3 Mol-% $Y_2O_3$ (IPS e.max ZirCAD LT A3 B45, Ivoclar Vivadent) bzw. aus kommerziell erhältlichen Oxidkeramik-Scheiben auf Basis von Zirkonoxid (Zenostar MT 3, Wieland Dental + Technik) erhaltene Prüfkörper wurden jeweils in einem Sinterofen mit $MoSi_2$-Heizelement gesintert. Dazu wurden die Prüfkörper bei Raumtemperatur in die Heizkammer des Sinterofens eingebracht, die Heizkammer verschlossen und gemäß Tabelle 3 in der Heizkammer ein partielles Vakuum mit einem finalen Druck von 50, 100 bzw. 200 mbar erzeugt. Die Prüfkörper wurden mit einer Heizrate von etwa 180 K/min auf eine Temperatur von etwa 1000°C und weiter mit einer Heizrate von etwa 80 K/min auf eine Temperatur von etwa 1350°C aufgeheizt. Bei Erreichen dieser Temperatur wurde die Heizkammer mit Frischluft geflutet und anschließend kontinuierlich mit einer Strömungsgeschwindigkeit von etwa 5 l/min mit Frischluft durchströmt, während die Prüfkörper mit einer Heizrate von etwa 80 K/min weiter auf eine Temperatur von 1500°C aufgeheizt, bei dieser Temperatur etwa 5 Minuten lang gehalten und dann mit einer Abkühlrate von etwa 140 K/min auf eine Temperatur von etwa 1100°C abgekühlt wurden. Anschließend wurde die Heizkammer geöffnet.

Die Dichte der jeweils erhaltenen Oxidkeramikmaterialien sind in Tabelle 3 wiedergegeben.

Tabelle 3

| Material | Partielles Vakuum [mbar] | Dichte [g/cm³] |
|---|---|---|
| IPS e.max ZirCAD LT A3 B45 | 50 | 6, 085 |
| | 100 | 6, 084 |
| | 200 | 6,081 |
| Zenostar MT A3 | 50 | 6, 052 |
| | 100 | 6, 049 |
| | 200 | 6, 048 |

**Beispiel 4**

Gemäß der allgemeinen Verfahrensweise aus kommerziell erhältlichen Oxidkeramik-Blöcken auf Basis von Zirkonoxid mit 3 Mol-% $Y_2O_3$ (IPS e.max ZirCAD LT BL B45 oder IPS e.max ZirCAD LT A3 B45, Ivoclar Vivadent) bzw. aus kommerziell erhältlichen Oxidkeramik-Scheiben auf Basis von Zirkonoxid (Zenostar MT 3, Wieland Dental + Technik) erhaltene Prüfkörper wurden jeweils in einem Sinterofen mit SiC-Heizelement gesintert. Dazu wurden die Prüfkörper bei Raumtemperatur in die Heizkammer des Sinterofens eingebracht, die Heizkammer verschlossen und in der Heizkammer ein partielles Vakuum mit einem finalen Druck von etwa 50 bis 100 mbar erzeugt. Die Prüfkörper wurden mit einer Heizrate von etwa 180 K/min auf eine Temperatur von etwa 1050°C und weiter mit einer Heizrate von etwa 80 K/min auf eine Temperatur von etwa 1500°C aufgeheizt. Gemäß Tabelle 4 wurde die Heizkammer bei Erreichen einer Temperatur von 1250, 1300 bzw. 1350°C mit Frischluft bzw. Sauerstoff geflutet und anschließend während des restlichen Sinterprozesses kontinuierlich mit einer Strömungsgeschwindigkeit von etwa 5 l/min durchströmt. Bei einer Temperatur von etwa 1500°C wurden die Prüfkörper etwa 5 Minuten lang gehalten und dann mit einer Abkühlrate von etwa 140 K/min auf eine Temperatur von etwa 1100°C abgekühlt. Anschließend wurde die Heizkammer geöffnet.

Zum Vergleich wurde die obige Verfahrensweise wiederholt, wobei jedoch kein Vakuum erzeugt wurde, sondern die Heizkammer über die gesamte Dauer des Sinterprozesses kontinuierlich mit einer Strömungsgeschwindigkeit von etwa 5 l/min gemäß Tabelle 4 mit Frischluft oder Sauerstoff durchströmt wurde. In einem weiteren Vergleich wurde die obige Verfahrensweise wiederholt, wobei jedoch das partielle Vakuum mit einem finalen Druck von etwa 50 bis 100 mbar über die gesamte Dauer des Sinterprozesses aufrechterhalten wurde.

Die Dichte, mittlere Korngröße, Transluzenz und Farbkoordinaten der jeweils erhaltenen Oxidkeramikmaterialien sind in Tabelle 4 wiedergegeben.

Tabelle 4

| Material | Vakuum bis [°C] | Atmosphäre | Dichte [g/cm³] | Mittl. Korngröße [µm]* | CR [%] | L* | a* | b* |
|---|---|---|---|---|---|---|---|---|
| IPS e.max ZirCAD LT BL B45 | 1350 | Luft | 6, 066 | 0,396 ± 0,075 | 88, 99 | 95,87 | -0,37 | 1,75 |
| | 1350 | O₂ | 6, 057 | 0,375 ± 0,061 | 91,27 | 95,96 | -0,27 | 1,83 |
| | durchgehend Luft* | | 6, 067 | 0,389 ± 0,077 | 92,54 | 96, 14 | -0,31 | 1, 81 |
| | durchgehend O₂* | | 6, 047 | 0,386 ± 0,062 | 95,11 | 96,59 | -0,21 | 1,57 |
| IPS e.max ZirCAD LT A3 B45 | 1300 | Luft | 6, 088 | | 97,57 | 83,58 | 4,34 | 17,13 |
| | durchgehend Luft* | | 6, 076 | | 98,34 | 83,72 | 4,23 | 17,02 |
| | durchgehend O₂* | | 6,065 | | 100 | 85,49 | 2,83 | 15,15 |
| Zenostar MT A3 | 1250 | Luft | 6,052 | 0,584 ± 0,103 | 96,54 | 83,38 | 2,46 | 15,94 |
| | 1250 | O₂ | 6,052 | 0,543 ± 0,108 | 96,77 | 82,50 | 2, 94 | 15,94 |
| | durchgehend Luft* | | 6,051 | 0,499 ± 0,131 | 98,31 | 84, 93 | 1,57 | 14, 04 |
| | durchgehend Vakuum* | | 6,051 | 0,528 ± 0,097 | 97, 64 | 82, 01 | 0,09 | 12,05 |

* (Vergleich)
** Die nach DIN EN 623-3 gemessene zahlenmittlere Korngröße wurde nach M. I. Mendelson, J. Am. Ceram. Soc. 1969, 52(8), 443-446 mit 1,56 multipliziert, um den realen zahlenmittleren Durchmesser im dreidimensionalen Mikrogefüge zu erhalten.

**Patentansprüche**

1. Verfahren zur Herstellung einer dentalen Restauration, bei dem ein Oxidkeramikmaterial

   (a) einer ersten Wärmebehandlung unterworfen wird,
   (b) einer zweiten Wärmebehandlung unterworfen wird und
   (c) abgekühlt wird,

   wobei die Wärmebehandlung in Schritt (a) bei niedrigerem Druck als die Wärmebehandlung in Schritt (b) erfolgt.

2. Verfahren nach Anspruch 1, bei dem in Schritt (a) das Oxidkeramikmaterial auf eine Temperatur aufgeheizt wird, die im Bereich von 1100 bis 1600°C, insbesondere im Bereich von 1200 bis 1500°C, bevorzugt im Bereich von 1250 bis 1450°C und weiter bevorzugt im Bereich von 1300 bis 1400°C liegt und am meisten bevorzugt etwa 1350°C beträgt.

3. Verfahren nach Anspruch 1 oder 2, bei dem das Oxidkeramikmaterial in Schritt (a) mit einer Heizrate im Bereich von 10 bis 500 K/min, insbesondere 50 bis 250 K/min und bevorzugt 100 bis 200 K/min aufgeheizt wird.

4. Verfahren nach einem der Ansprüche 1 bis 3, bei dem die Wärmebehandlung in Schritt (a) bei einem Druck von weniger als 200 mbar, vorzugsweise weniger als 100 mbar und besonders bevorzugt weniger als 50 mbar und insbesondere bei einem Druck im Bereich von 0,1 bis 200 mbar, vorzugsweise im Bereich von 1 bis 150 mbar und besonders bevorzugt im Bereich von 50 bis 100 mbar erfolgt.

5. Verfahren nach einem der Ansprüche 1 bis 4, bei dem in Schritt (b) das Oxidkeramikmaterial (b1) gegebenenfalls weiter aufgeheizt wird und (b2) bei einer, vorzugsweise konstanten, Temperatur im Bereich von 1100 bis 1700°C, insbesondere im Bereich von 1300 bis 1600°C und bevorzugt im Bereich von 1350 bis 1550°C und besonders bevorzugt bei einer Temperatur von etwa 1500°C gehalten und gesintert wird.

6. Verfahren nach Anspruch 5, bei dem das Halten in Schritt (b2) für 1 bis 60 Minuten, insbesondere 2 bis 30 Minuten, bevorzugt 3 bis 15 Minuten und besonders bevorzugt etwa 5 Minuten erfolgt.

7. Verfahren nach einem der Ansprüche 1 bis 6, bei dem die Wärmebehandlung in Schritt (b) bei einem Druck von mehr als 500 mbar und insbesondere bei Umgebungsdruck erfolgt.

8. Verfahren nach einem der Ansprüche 1 bis 7, bei dem die Wärmebehandlung in Schritt (b) in einer sauerstoffhaltigen Atmosphäre und insbesondere in Luft, mit Sauerstoff angereicherter Luft oder Sauerstoff erfolgt.

9. Verfahren nach Anspruch 8, bei dem während Schritt (b) die Heizkammer diskontinuierlich oder vorzugsweise kontinuierlich mit einer sauerstoffhaltigen Atmosphäre, vorzugsweise Luft, mit Sauerstoff angereicherter Luft oder Sauerstoff, durchströmt wird, insbesondere mit einer Strömungsgeschwindigkeit von 0,1 bis 50 l/min, bevorzugt 1 bis 20 l/min und besonders bevorzugt 4 bis 8 l/min.

10. Verfahren nach einem der Ansprüche 5 bis 9, bei dem das Oxidkeramikmaterial in Schritt (a) auf eine Temperatur aufgeheizt wird, die 0 bis 500 **K,** insbesondere 10 bis 250 K, bevorzugt 50 bis 200 K und besonders bevorzugt 100 bis 150 K unterhalb der Temperatur oder des Temperaturbereichs liegt, bei der/dem das Oxidkeramikmaterial in Schritt (b) gehalten wird.

11. Verfahren nach einem der Ansprüche 1 bis 10, bei dem das Oxidkeramikmaterial in Schritt (c) auf eine Temperatur abgekühlt wird, die im Bereich von 20 bis 1300°C, insbesondere im Bereich von 100 bis 1250°C und bevorzugt im Bereich von 1000 bis 1200°C liegt.

12. Verfahren nach einem der Ansprüche 1 bis 11, bei dem das Oxidkeramikmaterial auf Zirkonoxid und insbesondere auf polykristallinem tetragonalen Zirkonoxid (TZP) basiert.

13. Verfahren nach Anspruch 12, bei dem das Zirkonoxid mit $Y_2O_3$, $CeO_2$, MgO und/oder CaO stabilisiert ist und vorzugsweise mit 2 bis 12 Mol-%, insbesondere 3 bis 5 Mol-%, dieser Oxide, bezogen auf den Gehalt an Zirkonoxid, stabilisiert ist.

14. Verfahren nach einem der Ansprüche 1 bis 13, bei dem das Oxidkeramikmaterial eingefärbt ist und vorzugsweise mindestens zwei Schichten umfasst, die sich insbesondere in ihrer Farbe unterscheiden.

15. Verfahren nach einem der Ansprüche 1 bis 14, bei dem die dentale Restauration eine Brücke, ein Inlay, ein Onlay, eine Krone, ein Veneer, eine Schale oder ein Abutment ist und vorzugsweise zwei oder mehr Glieder umfasst.

16. Verwendung eines Oxidkeramikmaterials zur Herstellung einer dentalen Restauration, bei der das Oxidkeramikmaterial

    (a) einer ersten Wärmebehandlung unterworfen wird,
    (b) einer zweiten Wärmebehandlung unterworfen wird und
    (c) abgekühlt wird,

wobei die Wärmebehandlung in Schritt (a) bei niedrigerem Druck als die Wärmebehandlung in Schritt (b) erfolgt.

Europäisches
Patentamt

European
Patent Office

Office européen
des brevets

# EUROPÄISCHER RECHERCHENBERICHT

**Nummer der Anmeldung**

EP 24 22 3902

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 022 442 A1 (SEIKO EPSON CORP [JP]) 11. Februar 2009 (2009-02-11) | 1-7, 10-13, 15,16 | INV. A61C13/00 A61C13/083 |
| A | * Seite 2 - Seite 19; Abbildungen 1-6 * ----- | 8,9,14 | A61C5/77 |
| X | WO 2015/200017 A1 (3M INNOVATIVE PROPERTIES CO [US]) 30. Dezember 2015 (2015-12-30) | 1,3,7-9, 11,15,16 | |
| A | * Seite 1 - Seite 31; Abbildungen 1-8 * ----- | 2,4-6, 10,12-14 | |
| X | EP 0 853 938 A1 (INJEX CORP [JP]; MATSUMOTO DENTAL COLLEGE [JP]) 22. Juli 1998 (1998-07-22) | 1,4-6, 10-12, 14-16 | |
| A | * Seite 1 - Seite 18; Abbildungen 1-3 * ----- | 2,3,7-9, 13 | |

**RECHERCHIERTE SACHGEBIETE (IPC)**

A61C

Der vorliegende Recherchenbericht wurde für alle Patentansprüche erstellt

| Recherchenort | Abschlußdatum der Recherche | Prüfer |
|---|---|---|
| München | 5. Mai 2025 | Wirth, Christian |

EPO FORM 1503 03.82 (P04C03)

**EP 4 566 568 A1**

## ANHANG ZUM EUROPÄISCHEN RECHERCHENBERICHT
## ÜBER DIE EUROPÄISCHE PATENTANMELDUNG NR.

EP 24 22 3902

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-05-2025

| Im Recherchenbericht angeführtes Patentdokument | Datum der Veröffentlichung | Mitglied(er) der Patentfamilie | Datum der Veröffentlichung |
|---|---|---|---|
| EP 2022442 A1 | 11-02-2009 | CN 101352370 A | 28-01-2009 |
| | | CN 102614024 A | 01-08-2012 |
| | | EP 2022442 A1 | 11-02-2009 |
| | | JP 4321636 B2 | 26-08-2009 |
| | | JP 2009028359 A | 12-02-2009 |
| | | US 2009029321 A1 | 29-01-2009 |
| | | US 2013154140 A1 | 20-06-2013 |
| WO 2015200017 A1 | 30-12-2015 | EP 3157461 A1 | 26-04-2017 |
| | | US 2017128174 A1 | 11-05-2017 |
| | | US 2022125561 A1 | 28-04-2022 |
| | | US 2025114176 A1 | 10-04-2025 |
| | | WO 2015200017 A1 | 30-12-2015 |
| EP 0853938 A1 | 22-07-1998 | DE 69801010 T2 | 21-03-2002 |
| | | EP 0853938 A1 | 22-07-1998 |
| | | JP H10201771 A | 04-08-1998 |
| | | US 5984683 A | 16-11-1999 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2098188 A1
- EP 2101133 A1
- WO 2012057829 A2
- WO 2015091744 A1
- WO 2015121364 A1
- WO 2011020688 A1
- EP 2703760 A1

**In der Beschreibung aufgeführte Nicht-Patentliteratur**

- **M. I. MENDELSON**. *J. Am. Ceram. Soc.*, 1969, vol. 52 (8), 443-446